# EUROPÄISCHE PATENTANMELDUNG

(11) **EP 4 427 842 A1**
(43) Veröffentlichungstag der Anmeldung: **11.09.2024**
(21) Anmeldenummer: 24162395.8
(22) Anmeldetag: 08.03.2024
(51) Int. Cl.: B01L 3/02, B01L 3/00

(54) **PROBENENTNAHMEVORRICHTUNG, PROBENENTNAHMESYSTEM UND VER- FAHREN ZU EINEM BETRIEB EINER PROBENENTNAHMEVORRICHTUNG**

(30) Priorität: 10.03.2023 DE 102023106084; 16.05.2023 DE 102023112897
(71) Anmelder: Lockcon AG, 9500 Wil (CH)
(72) Erfinder: Oertli, Christian, 9621 Oberhelfenschwil (CH); Egli, Monika, 9500 Wil (CH)
(74) Vertreter: Daub, Thomas

(57) **Zusammenfassung**

Die Erfindung geht aus von einer Probenentnahmevorrichtung (10; 10a; 16b) mit einer Applikatoreinheit (12; 12a; 12b) zu einer Applikation einer Flüssigkeitsprobe auf eine erste Probenkarte (14; 14a; 14b).

Es wird vorgeschlagen, dass die Applikatoreinheit (12; 12a; 12b) in einem Applikationszustand zu einer gleichzeitigen Applikation der Flüssigkeitsprobe auf eine zweite Probenkarte (16; 16a; 16b) vorgesehen ist.

## Beschreibung

### Stand der Technik

Die Erfindung betrifft eine Probenentnahmevorrichtung nach dem Oberbegriff des Anspruchs 1, ein Probenentnahmesystem nach Anspruch 17 und ein Verfahren zu einem Betrieb einer Probenentnahmevorrichtung nach Anspruch 18.

Es sind bereits Vorrichtungen zu einer Applikation einer Flüssigkeitsprobe auf eine Probenkarte vorgeschlagen worden.

Die Aufgabe der Erfindung besteht insbesondere darin, eine gattungsgemäße Vorrichtung mit verbesserten Eigenschaften hinsichtlich einer Probenentnahmeeffizienz bereitzustellen. Die Aufgabe wird erfindungsgemäß durch die Merkmale der Patentansprüche 1 und 18 gelöst, während vorteilhafte Ausgestaltungen und Weiterbildungen der Erfindung den Unteransprüchen entnommen werden können.

### Vorteile der Erfindung

Die Erfindung geht aus von einer Probenentnahmevorrichtung mit einer Applikatoreinheit zu einer Applikation einer Flüssigkeitsprobe auf eine erste Probenkarte.

Es wird vorgeschlagen, dass die Applikatoreinheit in einem Applikationszustand zu einer gleichzeitigen Applikation der Flüssigkeitsprobe auf eine zweite Probenkarte vorgesehen ist.

Durch eine derartige Ausgestaltung der Probenentnahmevorrichtung kann eine Probenentnahmeeffizienz vorteilhaft gesteigert werden. Insbesondere kann ein zweiter Probenspot auf der zweiten Probenkarte gleichzeitig zu einem ersten Probenspot auf der ersten Probenkarte erzeugt werden, welche, insbesondere durch das Aufweisen der gleichen Flüssigkeitsprobe, zueinander vergleichbar sind. Ferner können die erste und die zweite Probenkarte getrennt voneinander transportiert und/oder gelagert werden. Insbesondere kann die Probenentnahmevorrichtung vorteilhaft, insbesondere gleichzeitig, eine "A"-Probe auf der ersten Probenkarte und eine "B"- Probe auf der zweiten Probenkarte bereitstellen, beispielsweise für eine Doping-Kontrolle.

Bei der Flüssigkeitsprobe handelt es sich vorzugsweise um eine Blutprobe. Die Flüssigkeitsprobe weist vorzugsweise Blut, insbesondere Kapillarblut, auf, wobei es sich bei dem Blut bevorzugt um Vollblut handelt. Alternativ oder zusätzlich ist denkbar, dass die Flüssigkeitsprobe Blutserum und/oder Blutplasma oder dergleichen aufweist. Die Flüssigkeitsprobe kann weitere Stoffe, beispielsweise ein Verdünnungsmittel oder dergleichen aufweisen. Alternativ oder zusätzlich wäre denkbar, dass die Flüssigkeitsprobe eine andere, insbesondere als Körperflüssigkeit ausgebildete, Flüssigkeit aufweist.

Insbesondere ist die Probenentnahmevorrichtung dazu vorgesehen, zumindest den ersten und den zweiten Probenspot, welche die Flüssigkeitsprobe zumindest teilweise aufweisen, auf der jeweiligen Probenkarte bereitzustellen und insbesondere aus der Flüssigkeitsprobe zu erzeugen. Die Probenentnahmevorrichtung ist insbesondere zu einer Verwendung in einem Dried Blood Spot-Test vorgesehen, wobei ein jeweiliger Probenspot in dem Dried Blood Spot-Test insbesondere zu einem Dried Blood Spot weiterverarbeitet, insbesondere getrocknet, werden kann.

Unter einer "gleichzeitigen Applikation" soll insbesondere eine Applikation einer Flüssigkeitsprobe, insbesondere auf die zweite Probenkarte verstanden werden, welche zumindest in einem selben Arbeitsvorgang und vorzugsweise zumindest im Wesentlichen zu einem gleichen Zeitpunkt durchgeführt wird, wie die Applikation der Flüssigkeitsprobe auf die erste Probenkarte. Vorzugsweise überlappt bei der gleichzeitigen Applikation zumindest ein Zeitpunkt der Applikation der Flüssigkeitsprobe auf die erste Probenkarte mit zumindest einem Zeitpunkt der Applikation der Flüssigkeitsprobe auf die zweite Probenkarte. Es ist denkbar, dass die gleichzeitigen Applikationen lediglich zu gleichen Zeitpunkten stattfinden. Grundsätzlich wäre aber auch denkbar, dass die gleichzeitigen Applikationen der Flüssigkeitsprobe auf die erste Probenkarte und auf die zweite Probenkarte zumindest teilweise zeitgleich nacheinander, aber in demselben Arbeitsvorgang erfolgen. Insbesondere sind die erste und die zweite Probenkarte in demselben Arbeitszustand bei der gleichzeitigen Applikation gleichzeitig in der Probenentnahmevorrichtung angeordnet.

Die Probenentnahmevorrichtung ist vorzugsweise ein Teil eines Probenentnahmesystems, welches neben der Probenentnahmevorrichtung insbesondere die erste Probenkarte und die zweiten Probenkarte aufweist. Die Probenkarten sind vorzugsweise als, insbesondere handelsübliche, Trockenblutkarten ausgebildet. Die erste und die zweite Probenkarte sind vorzugsweise zueinander identisch ausgebildet. Die folgende Beschreibung einer Probenkarte ist sowohl auf die erste, als auch auf die zweite Probenkarte zu lesen. Die Probenkarte weist vorzugsweise eine Kartenbasis, beispielsweise aus Pappe und/oder Papier auf. Insbesondere weist die Probenkarte zumindest einen, insbesondere aus Filterpapier ausgebildeten, Probenträger auf, welcher zu einer Absorption der Flüssigkeitsprobe zu einer Bereitstellung des Probenspots vorgesehen ist. Der Probenträger kann in einer rechteckigen oder kreisförmigen oder gemäß einer anderen, dem Fachmann sinnvoll erscheinenden Form geformten Ausnehmung der Kartenbasis angeordnet sein. Es ist denkbar, dass das Probenentnahmesystem weitere Einheiten und/oder Elemente, beispielsweise eine, insbesondere als Lanzette ausgebildete, Stecheinheit zu einer Punktion einer Testperson zu einer Kapillarblutentnahme, aufweist.

Unter "vorgesehen" soll ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Weiterhin wird vorgeschlagen, dass die Probenentnahmevorrichtung eine Kartenträgereinheit umfasst, welche eine erste Halterung für die erste Probenkarte und eine zweite Halterung für die zweite Probenkarte aufweist. Hierdurch können die erste und die zweite Probenkarte vorteilhaft für die Applikation der Flüssigkeitsprobe ausgerichtet und/oder bei der Applikation der Flüssigkeitsprobe in einer vorteilhaften Position gehaltert und insbesondere fixiert werden. Vorzugsweise sind die erste und die zweite Halterung identisch zueinander ausgebildet. Vorzugsweise fixiert die Halterung die Probenkarte an der Karteneinheit. Vorzugsweise ist eine Verbindung der Kartenträgereinheit und der jeweiligen Probenkarte mittels der jeweiligen zweiten Halterung trennbar ausgebildet. Die Halterung ist zu einer Halterung der Probenkarte vorzugsweise zu einer formschlüssigen Verbindung, insbesondere eine Steckverbindung und/oder eine Klemmverbindung, der Probenkarte und der Kartenträgereinheit, insbesondere der Halterung, vorgesehen. Die Halterung ist vorzugsweise zu einer formschlüssigen Aufnahme zumindest eines Teilbereichs der Probenkarte vorgesehen. Hierdurch kann die, insbesondere zumindest teilweise elastische verformbare, Probenkarte auf besonders einfache Weise an der Kartenträgereinheit angebracht und/oder von der Kartenträgereinheit entnommen werden.

Ferner wird vorgeschlagen, dass die Kartenträgereinheit ein erstes Kartenträgerelement, welches die erste Halterung aufweist, und ein relativ zu dem ersten Kartenträgerelement bewegbares zweites Kartenträgerelement, welches die zweite Halterung aufweist, umfasst. Hierdurch kann eine Position der an dem ersten Kartenträgerelement gehalterten ersten Probenkarte vorteilhaft bezüglich einer Position der an dem zweiten Kartenträgerelement gehalterten zweiten Probenkarte ausgerichtet werden. Insbesondere kann zumindest eine Entnahme und/oder eine Anbringung der ersten Probenkarte und/oder der zweiten Probenkarte von und/oder an der Kartenträgereinheit vorteilhaft vereinfacht werden.

Das erste Kartenträgerelement und das zweite Kartenträgerelement sind vorzugsweise identisch zueinander ausgebildet. Das jeweilige Kartenträgerelement ist vorzugsweise zumindest teilweise plattenartig ausgebildet und weist insbesondere eine Materialstärke auf, welche vorzugsweise maximal 50 %, bevorzugt höchstens 20 %, besonders bevorzugt maximal 10 %, und besonders vorteilhaft höchstens 5 %, einer Länge und/oder einer Breite des Kartenträgerelements entspricht, wobei das Kartenträgerelement insbesondere einander gegenüberliegende, Seiten aufweist, welche eine ebene Oberfläche aufweisen. Das erste Kartenträgerelement und/oder das zweite Kartenträgerelement weist/weisen vorzugsweise, insbesondere jeweils, ein Kartenträgerplattenelement auf, welches sich insbesondere entlang einer größten Seitenfläche des ersten Kartenträgerelements und/oder des zweiten Kartenträgerelements erstreckt. Vorzugsweise erstreckt sich die auf dem Kartenträgerelement gehalterte Probenkarte, insbesondere eine größte Seitenfläche der Probenkarte, parallel zu der größten Seitenfläche des Kartenträgerelements und/oder des Kartenträgerplattenelements. Die auf dem Kartenträgerelement gehalterte Probenkarte bildet vorzugsweise eine Kontaktfläche mit dem Kartenträgerplattenelement und/oder der größten Seitenfläche des Kartenträgerelements aus.

Es ist denkbar, dass die Halterung einen zumindest abschnittsweisen Überhang an einem Rand, insbesondere einer Ecke und/oder Kante, des Kartenträgerplattenelements aufweist, in welchem die Probenkarte eingeklemmt ist. Alternativ oder zusätzlich ist denkbar, dass die Halterung zumindest eine Rille und/oder Spalte zu einer zumindest teilweisen Aufnahme eines Randbereichs, insbesondere zumindest eines Kantenbereichs und/oder Eckbereichs, der Probenkarte aufweist. Es ist denkbar, dass die erste und/oder die zweite Probenkarte zumindest ein ausrichtungsanzeigendes Merkmal, wie beispielsweise eine abgerundete und oder abgeschnittene Ecke aufweist/aufweisen, anhand welchem insbesondere eine Ausrichtung der Probenkarte für die Halterung an der Kartenträgereinheit festgelegt ist.

Es wäre denkbar, dass das zweite Kartenträgerelement linear und/oder frei relativ zu dem zweiten Kartenträgerelement beweglich ist. In einer vorteilhaften Ausgestaltung der Kartenträgereinheit wird jedoch vorgeschlagen, dass das zweite Kartenträgerelement relativ zu dem ersten Kartenträgerelement mittels zumindest eines Scharniers der Kartenträgereinheit verschwenkbar ist. Hierdurch können besonders vorteilhafte relative Positionen des ersten Kartenträgerelements und des zweiten Kartenträgerelements erreicht werden. Es ist denkbar, dass die Kartenträgereinheit mehr als ein Scharnier, insbesondere zwei Scharniere, zu einer Verschwenkung des zweiten Kartenträgerelements relativ zu dem ersten Kartenträgerelement aufweist. Alternativ wäre denkbar, dass das zweite Kartenträgerelement unbeweglich relativ zu dem ersten Kartenträgerelement ist.

Weiterhin wird vorgeschlagen, dass das erste Kartenträgerelement und das zweite Kartenträgerelement fest miteinander verbunden sind. Hierdurch kann eine Komplexität der Kartenträgereinheit vorteilhaft reduziert und/oder ein Bedienkomfort gesteigert werden, insbesondere indem eine Anzahl von losen Bestandteilen der Kartenträgereinheit vorteilhaft reduziert werden kann. Insbesondere sind das erste Kartenträgerelement und das zweite Kartenträgerelement über das Scharnier fest miteinander verbunden. Bei der festen Verbindung handelt es sich insbesondere um eine, insbesondere durch den Bediener, zumindest teilweise untrennbare Verbindung und/oder um eine Verbindung, welche insbesondere in jedem Betriebszustand der Probenentnahmevorrichtung vorliegt. Das Scharnier ist vorzugsweise jeweils an einer, insbesondere zueinander identisch ausgebildeten, längsten Kante des ersten Kartenträgerelements und/oder des zweiten Kartenträgerelements angeordnet und insbesondere mit der jeweiligen längsten Kante verbunden. Das Scharnier ist insbesondere jeweils an dem Kartenträgerplattenelement des ersten Kartenträgerelements und/oder des zweiten Kartenträgerelements angeordnet und insbesondere mit dem jeweiligen Kartenträgerplattenelement verbunden. Die Verschwenkung weist vorzugsweise eine Rotationsachse parallel zu der längsten Kante des ersten Kartenträgerelements und/oder des zweiten Kartenträgerelements auf.

Es wäre denkbar, dass das Scharnier, insbesondere jeweils, über eine Klebe- und/oder Schraub- und/oder Steckverbindung und/oder dergleichen mit dem ersten Kartenträgerelement und/oder dem zweiten Kartenträgerelement verbunden ist. Vorzugsweise sind das erste Kartenträgerelement und das zweite Kartenträgerelement einteilig miteinander verbunden. Hierdurch kann insbesondere eine Komplexität der Kartenträgereinheit besonders vorteilhaft reduziert und/oder eine Herstellungseffizienz gesteigert werden. Unter "einteilig" soll insbesondere in einem Stück geformt verstanden werden. Vorzugsweise wird dieses eine Stück aus einem einzelnen Rohling, einer Masse und/oder einem Guss, besonders bevorzugt in einem Spritzgussverfahren, insbesondere einem Ein- und/oder Mehrkomponenten-Spritzgussverfahren, hergestellt. Das Scharnier ist vorzugsweise als ein Filmscharnier ausgebildet. Insbesondere ist das Scharnier einteilig mit dem ersten Kartenträgerelement und/oder dem zweiten Kartenträgerelement verbunden. Vorzugsweise ist die Kartenträgereinheit komplett einteilig ausgebildet. Vorzugsweise sind das erste Kartenträgerelement und das zweite Kartenträgerelement lediglich über das Scharnier einteilig miteinander verbunden. Alternativ oder zusätzlich ist denkbar, dass das erste Kartenträgerelement über die Applikatoreinheit mit dem zweiten Kartenträgerelement verbunden ist. Insbesondere ist denkbar, dass das erste Kartenträgerelement über zumindest ein erstes Scharnier mit der Applikatoreinheit verbunden ist und/oder dass das zweite Kartenträgerelement über zumindest ein zweites Scharnier mit der Applikatoreinheit verbunden ist. Die Kartenträgereinheit weist vorzugsweise zumindest einen Kunststoff auf und ist insbesondere aus Kunststoff ausgebildet. Die Kartenträgereinheit kann transparent oder zumindest teilweise opak ausgebildet sein.

Ferner wird vorgeschlagen, dass die Applikatoreinheit zumindest in dem Applikationszustand mit der Kartenträgereinheit verbunden ist, wodurch insbesondere eine Flüssigkeitsprobe vorteilhaft ausgehend von der Applikatoreinheit auf die in der jeweiligen Kartenträgereinheit gehalterte Probenkarte appliziert werden kann. Vorzugsweise sind die Applikatoreinheit und die Kartenträgereinheit zumindest in dem Applikationszustand unbeweglich relativ zueinander miteinander verbunden. Vorteilhaft kann eine besonders stabile Applikation und/oder eine besonders präzise Applikation, insbesondere auf den Probenträger der ersten Probenkarte und/oder der zweiten Probenkarte, bereitgestellt werden.

Zudem wird vorgeschlagen, dass die Applikatoreinheit in dem Applikationszustand zumindest teilweise zwischen dem ersten Kartenträgerelement und dem zweiten Kartenträgerelement angeordnet ist. Hierdurch kann die Probenentnahmevorrichtung besonders kompakt für die gleichzeitige Applikation der Flüssigkeitsprobe auf die erste Probenkarte und die zweite Probenkarte bereitgestellt werden. Insbesondere ist die erste Probenkarte zumindest in dem Applikationszustand zumindest teilweise zwischen der Applikatoreinheit und dem ersten Kartenträgerelement angeordnet. Insbesondere ist die zweite Probenkarte zumindest in dem Applikationszustand zumindest teilweise zwischen der Applikatoreinheit und dem zweiten Kartenträgerelement angeordnet. Insbesondere ist das erste Kartenträgerelement auf einer zu einer Anordnung des zweiten Kartenträgerelements gegenüberliegenden Seite der Applikatoreinheit angeordnet. Vorzugsweise sind die Applikatoreinheit, das erste Kartenträgerplattenelement und das zweite Kartenträgerplattenelement in dem Applikationszustand parallel zueinander angeordnet. Insbesondere erstrecken sich die größten Seitenflächen der Applikatoreinheit, des ersten Kartenträgerelements und des zweiten Kartenträgerelements in dem Applikationszustand parallel zueinander. Die Applikatoreinheit ist in dem Applikationszustand vorzugsweise zumindest zu einem Großteil zwischen dem ersten Kartenträgerelement und dem zweiten Kartenträgerelement angeordnet, wobei insbesondere zumindest 55 %, vorteilhaft zumindest 65 %, vorzugsweise zumindest 75 %, besonders bevorzugt zumindest 85 % und besonders vorteilhaft zumindest 90 % eines Volumens und/oder einer Masse der Applikatoreinheit zwischen dem ersten Kartenträgerelement und dem zweiten Kartenträgerelement angeordnet sind.

Vorzugsweise kann die Kartenträgereinheit zumindest zwischen dem Entnahmezustand und dem Applikationszustand, insbesondere relativ zu der Applikatoreinheit, verschwenkt werden.

Weiterhin wird vorgeschlagen, dass die Probenentnahmevorrichtung eine Fixiereinheit umfasst, welche ein Herausrutschen der Applikatoreinheit aus der Kartenträgereinheit verhindert. Hierdurch kann die Applikatoreinheit vorteilhaft stabil mit der Kartenträgereinheit verbunden werden. Insbesondere kann ein Verrutschen der Applikatoreinheit in dem Applikationszustand bezüglich der auf der Applikatoreinheit gehalterten ersten Probenkarte und/oder der zweiten Probenkarte vorteilhaft vermieden werden.

Die Fixiereinheit bildet vorzugsweise eine formschlüssige Verbindung, insbesondere eine Klemm- und/oder Schnappverbindung, zwischen der Applikatoreinheit und der Kartenträgereinheit aus. Die Fixiereinheit weist vorzugsweise zumindest ein Applikatorhalterungselement, insbesondere vier zueinander identisch ausgebildete Applikatorhalterungselemente an der Applikatoreinheit auf. Das Applikatorhalterungselement ist vorzugsweise Teil der Applikatoreinheit. Das Applikatorhalterungselement bildet vorzugsweise zumindest eine Stützfläche für eine Halterung der Applikatoreinheit aus. Die Fixiereinheit weist vorzugsweise einen in dem Applikatorhalterungselement aufgenommenen Randbereich der Kartenträgereinheit, insbesondere eines Kartenträgerelements, auf. Das Applikatorhalterungselement weist vorzugsweise einen Knick zu der Aufnahme des Randbereichs der Kartenträgereinheit auf. Der Knick kann insbesondere einen Winkel von zumindest im Wesentlichen 90° aufweisen, wobei der Winkel insbesondere weniger als 20°, vorzugsweise weniger als 10° und besonders bevorzugt weniger als 5° von einem Winkel von 90° abweicht. Es ist denkbar, dass das Applikatorhalterungselement, insbesondere zumindest zu einer vereinfachten Einführung und/oder Herausnahme der Kartenträgereinheit und/oder zu der Verschwenkung zumindest teilweise elastisch und/oder klappbar ausgebildet ist. Es ist denkbar, dass das Applikatorhalterungselement eine Verschwenkung des zweiten Kartenträgerelements relativ zu dem ersten Kartenträgerelement in dem Applikationszustand begrenzt und/oder verhindert. Alternativ oder zusätzlich ist denkbar, dass die Fixiereinheit einstückig mit dem Scharnier ausgebildet ist.

Die Applikatoreinheit ist insbesondere in einem montierten Zustand mittels der Fixiereinheit mit der Kartenträgereinheit verbunden. Vorzugsweise umfasst der montierte Zustand zumindest den Applikationszustand und insbesondere einen Entnahmezustand.

Ferner wird vorgeschlagen, dass die Probenentnahmevorrichtung eine Verschlusseinheit aufweist, welche eine relative Lage des ersten Kartenträgerelements zu dem zweiten Kartenträgerelement in dem Applikationszustand fixiert. Hierdurch kann eine vorteilhafte und insbesondere stabile Position der Probenkarten in dem Applikationszustand bereitgestellt werden.

Die Verschlusseinheit bildet vorzugsweise eine formschlüssige Verbindung, beispielsweise eine Klemmverbindung und/oder vorteilhaft eine Schnappverbindung, zwischen der Applikatoreinheit und der Kartenträgereinheit und/oder zwischen dem ersten Kartenträgerelement und dem zweiten Kartenträgerelement aus. Die Verschlusseinheit weist vorzugsweise zumindest ein Verschlusselement, vorzugsweise vier zueinander identisch ausgebildete Verschlusselemente an der Applikatoreinheit auf. Das Verschlusselement ist vorzugsweise Teil der Applikatoreinheit. Das Verschlusselement weist vorzugsweise zumindest eine Ausnehmung auf, welche insbesondere zu einer Fixierung der relativen Lage des ersten Kartenträgerelements zu dem zweiten Kartenträgerelement in dem Applikationszustand zu zumindest einer teilweisen Aufnahme des Gegenverschlusselements vorgesehen ist. Vorzugsweise wirkt das Verschlusselement in dem Applikationszustand eine Kraft entlang einer Verschwenkrichtung des Kartenträgerelements auf das Gegenverschlusselement aus. Vorzugsweise sind das Verschlusselement und das Gegenverschlusselement für die Schnappverbindung zumindest teilweise elastisch ausgebildet. Das Gegenverschlusselement ist vorzugsweise Teil der Kartenträgereinheit. Vorteilhaft kann das Gegenverschlusselement als eine Unebenheit auf dem Kartenträgerelement, insbesondere an einer Kante des Kartenträgerelements, ausgebildet sein. Alternativ ist denkbar, dass das Verschlusselement und das Gegenverschlusselement Teil der Kartenträgereinheit sind, wobei diese zur Fixierung der relativen Lage des ersten Kartenträgerelements zu dem zweiten Kartenträgerelement in dem Applikationszustand insbesondere jeweils an dem ersten Kartenträgerelement und dem zweiten Kartenträgerelement angeordnet sind.

Vorzugsweise ist die Applikatoreinheit zumindest in dem Applikationszustand mittels der Fixiereinheit und/oder der Verschlusseinheit, insbesondere unbeweglich, mit der Kartenträgereinheit verbunden. Insbesondere sind das erste Kartenträgerelement und das zweite Kartenträgerelement und die Applikatoreinheit durch die Fixiereinheit und die Verschlusseinheit relativ zueinander fixiert.

Zudem wird vorgeschlagen, dass die Applikatoreinheit ein erstes Applikatorelement zu einer Applikation der Flüssigkeitsprobe auf die erste Probenkarte und ein zweites Applikatorelement zu einer Applikation der Flüssigkeitsprobe auf die zweite Probenkarte aufweist, wobei das erste Applikatorelement und das zweite Applikatorelement über eine Trenneinheit der Applikatoreinheit verbunden sind. Hierdurch kann eine gleichzeitige Applikation der Flüssigkeitsprobe auf die erste und die zweite Probenkarte besonders effektiv bereitgestellt werden. Weiterhin können die Applikatoreinheit und/oder die Probenentnahmevorrichtung besonders kompakt ausgebildet werden und/oder eine Herstellungseffizienz der Applikatoreinheit und/oder die Probenentnahmevorrichtung vorteilhaft gesteigert werden.

Eine jeweiliges Applikatorelement weist vorzugsweise zumindest das Applikatorhalterungselement und/oder das Verschlusselement und/oder ein Applikatorplattenelement auf. Insbesondere ist das Applikatorelement zumindest teilweise plattenartig ausgebildet.

Das Applikatorhalterungselement ist vorzugsweise mit dem Applikatorplattenelement verbunden. Das Applikatorhalterungselement kann mittels einer Schraub- und/oder Klebe- und/oder Steckverbindung oder dergleichen mit dem Applikatorplattenelement verbunden sein. Das Applikatorhalterungselement ist vorzugsweise einteilig mit dem Applikatorplattenelement verbunden. Das Applikatorhalterungselement ist vorzugsweise an einer zu der Trenneinheit gegenüberliegenden Seite des Applikatorplattenelements angeordnet.

Das Verschlusselement ist vorzugsweise mit dem Applikatorplattenelement verbunden. Das Verschlusselement kann mittels einer Schraub- und/oder Klebe- und/oder Steckverbindung oder dergleichen mit dem Applikatorplattenelement verbunden sein. Das Verschlusselement ist vorzugsweise einteilig mit dem Applikatorplattenelement verbunden. Das Verschlusselement erstreckt sich vorzugsweise oberhalb einer zu der Trenneinheit gegenüberliegenden Seite des Applikatorplattenelements. Das Verschlusselement ist vorzugsweise auf einer Oberseite, insbesondere bezüglich der Ausrichtung der Probenentnahmevorrichtung in zumindest dem Applikationszustand, des Applikatorplattenelements angeordnet. Das Verschlusselement ist vorzugsweise oberhalb des Applikatorhalterungselements an dem Applikatorplattenelement angeordnet. Das Verschlusselement und das Applikatorhalterungselement weisen vorzugsweise zueinander parallele Flächen auf, welche sich insbesondere senkrecht zu der größten Seitenfläche des Applikatorelements erstrecken.

Vorzugsweise weist die Applikatoreinheit eine Verbindung zwischen einer größten Seitenfläche des ersten Applikatorelements und einer größten Seitenfläche eines zweiten Applikatorelements auf. Insbesondere ist das erste Applikatorplattenelement mit dem zweiten Applikatorplattenelement verbunden.

Die Trenneinheit weist insbesondere zumindest eine Teilfläche einer zu einer Verbindung vorgesehenen Seitenfläche des ersten und/oder zweiten Applikatorelements auf. Die Trenneinheit ist vorzugsweise als eine Folieneinheit ausgebildet, welche insbesondere zumindest eine Folie aufweist. Die Folieneinheit kann mehr als eine Folie aufweisen, welche insbesondere zumindest teilweise aufeinander oder versetzt zueinander zwischen dem ersten Applikatorelement und/oder dem zweiten Applikatorelement, angeordnet sein können. Die Folie kann vorzugsweise als eine Klebefolie, insbesondere mit einem einseitigen und/oder zweiseitigen Klebemittel, ausgebildet sein. Alternativ kann die Folie beispielsweise als Adhäsionsfolie oder Haftfolie oder dergleichen ausgebildet sein.

Alternativ und insbesondere zusätzlich ist denkbar, dass das erste Applikatorelement und das zweite Applikatorelement über einen anderen Verbindungsmechanismus verbunden sind. Insbesondere ist denkbar, dass das erste Applikatorelement und das zweite Applikatorelement zumindest über eine formschlüssige und/oder reibschlüssige Verbindung, insbesondere über zumindest eine Steckverbindung, verbunden sind. Vorzugsweise weisen das erste Applikatorelement und das zweite Applikatorelement zumindest eine Ausnehmung und/oder zumindest ein Steckelement zu einer Ausbildung der Steckverbindung auf, wobei die Ausnehmung des ersten Applikatorelements insbesondere zu einer Aufnahme eines Steckelements des zweiten Applikatorelements vorgesehen ist und/oder das Steckelement des ersten Applikatorelements insbesondere zu einer Aufnahme in der Ausnehmung des zweiten Applikatorelements vorgesehen ist. Alternativ oder zusätzlich wäre denkbar, dass die Applikatoreinheit eine andere Verbindung des ersten Applikatorelements und des zweiten Applikatorelements, beispielsweise eine Schraubverbindung und/oder eine Schweißverbindung und/oder eine andere, dem Fachmann sinnvoll erscheinende Verbindung, aufweist.

Weiterhin wird vorgeschlagen, dass das erste Applikatorelement zumindest einen ersten Strömungskanal und das zweite Applikatorelement zumindest einen zweiten Strömungskanal aufweist, wobei der erste Strömungskanal und der zweite Strömungskanal durch die Trenneinheit zumindest teilweise verschlossen sind. Hierdurch kann die gleichzeitige Applikation der Flüssigkeitsprobe besonders vorteilhaft bereitgestellt werden. Insbesondere kann eine Herstellungseffizienz, insbesondere bezüglich einer Ausbildung der Strömungskanäle, vorteilhaft gesteigert werden.

Der erste Strömungskanal und der zweite Strömungskanal sind in dem ersten Applikatorelement und dem zweiten Applikatorelement vorzugsweise identisch zueinander ausgebildet. Der erste Strömungskanal und/oder der zweite Strömungskanal ist vorzugsweise als eine durch die Trenneinheit, insbesondere die Folieneinheit, zumindest teilweise verschlossene Fräsung in dem ersten Applikatorelement und/oder dem zweiten Applikatorelement, insbesondere in dem ersten und/oder zweiten Applikatorplattenelement, ausgebildet. Insbesondere sind der erste Strömungskanal und der zweite Strömungskanal durch die Trenneinheit entlang einer Längserstreckungsrichtung des jeweiligen Strömungskanals verschlossen. In einer Ausgestaltung der Folieneinheit als zumindest eine Klebefolie ist denkbar, dass die Klebefolie eine Aussparung des Klebemittels, zumindest in einem Bereich des ersten Strömungskanals und/oder des zweiten Strömungskanals aufweist, wodurch insbesondere zumindest eine Kontamination des ersten Strömungskanals und/oder des zweiten Strömungskanals vorteilhaft vermieden und/oder reduziert werden kann.

Der erste Strömungskanal und der zweite Strömungskanal sind insbesondere zumindest zu einem Transport der Flüssigkeitsprobe zu der Applikation auf die erste Kartenträgereinheit bzw. die zweite Kartenträgereinheit vorgesehen. Hierdurch kann vorteilhaft ein Bedienkomfort gesteigert werden, insbesondere indem eine Flüssigkeitsquelle, beispielsweise ein eingestochener Finger oder dergleichen, nicht direkt auf die Probenkarte aufgelegt werden muss. Insbesondere kann die Probenentnahmevorrichtung vorteilhaft für eine Heimanwendung bereitgestellt werden. Der erste Strömungskanal und/oder der zweite Strömungskanal weist/weisen, insbesondere jeweils, einen Kanaleingang und/oder einen Kanalausgang auf. Der Kanaleingang ist insbesondere zu einer Aufnahme der Flüssigkeitsprobe vorgesehen. Insbesondere kann der Kanaleingang zu einem Auflegen der Flüssigkeitsquelle der Flüssigkeitsprobe vorgesehen sein. Der erste Strömungskanal und/oder der zweite Strömungskanal weisen/weisen insbesondere jeweils einen bei einer korrekten Positionierung der Probenentnahmevorrichtung, insbesondere zumindest bezüglich einer Schwerkraftrichtung in dem Aufnahmezustand und/oder dem Applikationszustand, unterhalb dem Kanaleingang angeordneten Kanalausgang auf. Hierdurch kann die Flüssigkeitsprobe insbesondere zumindest teilweise mittels einer Schwerkraft von dem Kanaleingang zu dem Kanalausgang transportiert werden Der Kanaleingang ist vorzugsweise an einer oberen Seitenkante des Applikatorelements angeordnet. Der Kanaleingang des ersten Strömungskanals und der Kanaleingang des zweiten Strömungskanals in der Applikatoreinheit sind vorzugsweise zu einer gleichzeitigen Entnahme der Flüssigkeitsprobe von der Flüssigkeitsquelle vorgesehen. Vorzugsweise sind der Kanaleingang des ersten Strömungskanals und der Kanaleingang des zweiten Strömungskanals lediglich durch die Trenneinheit, insbesondere lediglich die Folieneinheit, voneinander beabstandet, wodurch eine besonders vorteilhafte gleichzeitige Aufnahme der Flüssigkeitsprobe in den ersten Strömungskanal und den zweiten Strömungskanal bereitgestellt werden kann. Der erste Strömungskanal und/oder der zweite Strömungskanal ist/sind vorzugsweise als Kapillare/n ausgebildet, wobei die Flüssigkeitsprobe insbesondere zumindest teilweise über eine Kapillarwirkung von dem Kanaleingang zu dem Kanalausgang transportiert wird. Der Kanalausgang kann eine Verjüngung eines Durchmessers des Strömungskanals aufweisen, wodurch insbesondere ein Ausfluss der Flüssigkeitsprobe außerhalb des Applikationszustands vorteilhaft vermieden werden kann. In dem Applikationszustand steht der Kanalausgang vorzugsweise in Kontakt mit dem Probenträger der jeweiligen Probenkarte, wobei die Flüssigkeitsprobe zumindest teilweise über eine Saugleistung des Probenträgers von dem Kanalausgang zu dem Probenträger transportiert werden kann. Der Kanalausgang des ersten Strömungskanals ist vorzugsweise auf einer zu dem Kanalausgang des zweiten Strömungskanals gegenüberliegenden, insbesondere größten, Seitenfläche der Applikatoreinheit angeordnet. Der Kanalausgang ist vorzugsweise in einer Ausbuchtung des Applikatorelements, insbesondere des Applikatorplattenelements, ausgebildet, wodurch insbesondere ein Kontakt des Kanalausgangs und der Probenkarte, insbesondere dem Probenträger, zuverlässig bereitgestellt werden kann.

Der erste Strömungskanal und/oder der zweite Strömungskanal ist/sind vorzugsweise volumenmessend ausgebildet, wobei insbesondere ein mit der Flüssigkeitsprobe befülltes quantifiziertes Volumen des ersten Strömungskanals und/oder des zweiten Strömungskanals auf die erste Probenkarte und/oder die zweite Probenkarte appliziert wird/werden. Vorzugsweise ist/sind der erste Strömungskanal und/oder der zweite Strömungskanal zu einer Aufbewahrung der Flüssigkeitsprobe bis zu der Erreichung des Applikationszustands vorgesehen. Vorzugsweise ist/sind der erste Strömungskanal und/oder der zweite Strömungskanal dazu vorgesehen, komplett und/oder bis zu einer Markierung befüllt zu werden. Es ist denkbar, dass die Applikatoreinheit zumindest teilweise transparent ausgebildet ist, wodurch die Befüllung des ersten Strömungskanals und/oder des zweiten Strömungskanals durch den Bediener vorteilhaft vereinfacht werden kann. Vorzugsweise weisen der erste Strömungskanal und/oder der zweite Strömungskanal ein Volumen von zumindest 5 µL, bevorzugt zumindest 10 µL, besonders bevorzugt zumindest 15 µL und/oder maximal 40 µL, bevorzugt maximal 30 µL, besonders bevorzugt maximal 25 µL auf. Besonders vorteilhaft weisen der erste Strömungskanal und/oder der zweite Strömungskanal ein Volumen von genau 20 µL auf. Vorzugsweise weisen der erste Strömungskanal und der zweite Strömungskanal ein gleiches Volumen auf. Der erste Strömungskanal und/oder der zweite Strömungskanal ist/sind insbesondere gekrümmt ausgebildet, wobei ein vorteilhaftes Volumen des ersten Strömungskanals und/oder des zweiten Strömungskanals und eine besonders kompakte Ausgestaltung der Applikatoreinheit bereitgestellt werden kann. Insbesondere kann ein Auslaufen der Flüssigkeitsprobe aus dem ersten Strömungskanal und/oder dem zweiten Strömungskanal außerhalb des Applikationszustands vorteilhaft vermieden oder reduziert werden.

Vorzugsweise weisen/weist das erste Applikatorelement und/oder das zweite Applikatorelement zumindest einen weiteren ersten Strömungskanal und/oder zumindest einen weiteren zweiten Strömungskanal auf. Hierdurch kann eine Entnahmeeffizienz vorteilhaft gesteigert werden und insbesondere mehr als ein Probenspot auf der ersten Probenkarte und/oder der zweiten Probenkarte bereitgestellt werden. Beispielsweise kann/können das erste Applikatorelement und/oder das zweite Applikatorelement, insbesondere jeweils, genau drei Strömungskanäle aufweisen. Vorzugsweise weisen alle Strömungskanäle der Applikatoreinheit ein jeweils zueinander identisches Volumen auf. Der Kanaleingang des Strömungskanals und ein Kanaleingang des weiteren Strömungskanals des jeweiligen Applikatorelements sind in der Applikatoreinheit vorzugsweise zu einer gleichzeitigen Entnahme der Flüssigkeitsprobe von der Flüssigkeitsquelle vorgesehen.

Ferner wird vorgeschlagen, dass das erste Applikatorelement und das zweite Applikatorelement zueinander identisch ausgebildet sind. Hierdurch kann insbesondere eine Herstellungseffizienz der Applikatoreinheit vorteilhaft gesteigert werden. Vorzugsweise sind gegenüberliegende Seiten des ersten Applikatorelements und des in der Applikatoreinheit mit dem ersten Applikatorelement verbundenen zweiten Applikatorelements zueinander identisch ausgebildet. Das erste Applikatorelement und das zweite Applikatorelement weisen vorzugsweise zumindest einen Kunststoff auf und können insbesondere aus Kunststoff ausgebildet sein. Alternativ wäre denkbar, dass das erste Applikatorelement und das zweite Applikatorelement zumindest teilweise unterschiedlich, beispielsweise spiegelverkehrt, zueinander ausgebildet sind.

In einem Aspekt der Erfindung, welcher für sich alleine genommen oder auch in Kombination mit weiteren Aspekten der Erfindung betrachtet werden kann, wird vorgeschlagen, dass die Probenentnahmevorrichtung eine Transporteinheit aufweist, welche zu einer Übertragung der Flüssigkeitsprobe an die Applikatoreinheit mit dieser verbindbar ist. Hierdurch kann eine besonders effektive und/oder einfache und/oder sichere Flüssigkeitsprobenentnahme, insbesondere Blutentnahme, erreicht werden. Vorteilhaft kann die Flüssigkeitsprobe an der Flüssigkeitsquelle besonders einfach und/oder effektiv entnommen und insbesondere auf eine besonders sichere Weise in die Applikatoreinheit eingespeist werden. Ferner kann eine Flüssigkeitsprobenentnahme an einer Körperstelle, in welcher die Flüssigkeitsprobenentnahme durch einen direkten Kontakt mit der Applikatoreinheit insbesondere besonders schwer umzusetzen ist, beispielsweise an einem Arm und/oder Fuß und/oder dergleichen, vorteilhaft bereitgestellt werden. Weiterhin kann die Flüssigkeitsprobe vorteilhaft zeitversetzt von der Flüssigkeitsquelle entnommen und in die Applikatoreinheit eingespeist werden.

Die Transporteinheit ist insbesondere zu einem Transport der Flüssigkeitsprobe zwischen der Flüssigkeitsquelle und der Applikatoreinheit vorgesehen. Die Transporteinheit weist insbesondere einen Aufnahmebereich zur Aufnahme der Flüssigkeitsprobe auf. Der Aufnahmebereich ist vorzugsweise zumindest zu einem Transport und/oder einer Lagerung der Flüssigkeitsprobe zumindest teilweise verschließbar. Vorzugsweise ist die Transporteinheit zumindest abschnittsweise röhrenförmig ausgebildet und der Aufnahmebereich ist insbesondere zumindest abschnittsweise zylinderförmig ausgebildet. Alternativ wäre eine andere dem Fachmann sinnvoll erscheinende Form der Transporteinheit und/oder des Aufnahmebereichs denkbar. Vorzugsweise weist die Transporteinheit einen, insbesondere zumindest abschnittsweise röhrenförmigen, Außenkörper auf, welcher den Aufnahmebereich zumindest teilweise begrenzt. Vorzugsweise weist die Transporteinheit, insbesondere an einer Oberseite der Transporteinheit, eine Aufnahmeöffnung zur Aufnahme der Flüssigkeitsprobe von der Flüssigkeitsquelle auf. Die Transporteinheit weist, insbesondere unterhalb der Aufnahmeöffnung, vorzugsweise zumindest eine Übertragungsöffnung zur Übertragung der Flüssigkeitsprobe an die Applikatoreinheit auf.

Insbesondere kann die Transporteinheit zumindest teilweise und insbesondere auch vollständig transparent ausgebildet sein, um einen Einblick in den Aufnahmebereich zu ermöglichen. Ferner kann die Transporteinheit eine Volumenanzeige, insbesondere eine Mindestvolumenanzeige, aufweisen, um ein Volumen, insbesondere ein Mindestvolumen, von Flüssigkeit um Aufnahmebereich anzuzeigen. Besonders bevorzugt kann das Mindestvolumen 120 µl betragen.

Die Transporteinheit ist vorzugsweise formschlüssig und/oder reibschlüssig mit der Applikatoreinheit verbindbar. Bevorzugt ist die Transporteinheit durch eine Steckverbindung mit der Applikatoreinheit verbindbar. Vorzugsweise ist die Transporteinheit zu einer Aufnahme des zumindest einen Kanaleingangs der Applikatoreinheit vorgesehen. Bevorzugt ist die Transporteinheit zu einer Aufnahme aller Kanaleingänge der Applikatoreinheit vorgesehen. Vorzugsweise ist die mit der Applikatoreinheit verbundene Transporteinheit zumindest zu einem Großteil, insbesondere zu zumindest 55 %, vorteilhaft zumindest 65 %, vorzugsweise zumindest 75 %, besonders bevorzugt zumindest 85 % und besonders vorteilhaft zumindest 90 % eines Volumens und/oder einer Masse der Transporteinheit, oberhalb der Applikatoreinheit angeordnet. Vorzugsweise ist der Aufnahmebereich oberhalb der Applikatoreinheit und insbesondere oberhalb dem zumindest einen Kanaleingang angeordnet. Hierdurch kann eine besonders einfache Übertragung der Flüssigkeitsprobe von der Transporteinheit an die Applikatoreinheit, insbesondere mittels Schwerkraft, bereitgestellt werden.

Weiterhin wird vorgeschlagen, dass die Transporteinheit eine Ventileinheit aufweist, welche dazu vorgesehen ist, beim Verbinden der Transporteinheit mit der Applikatoreinheit durch die Applikatoreinheit aufgedrückt zu werden. Hierdurch kann eine Verbindung zwischen der Transporteinheit und der Applikatoreinheit zu einem Übertrag der Flüssigkeitsprobe auf besonders vorteilhafte Weise bereitgestellt werden. Insbesondere kann eine Verbindung zum Flüssigkeitsprobenübertrag bei der Verbindung der Transporteinheit mit der Applikatoreinheit auf besonders einfache Weise automatisch und insbesondere ohne weitere Schritte bereitgestellt werden. Weiterhin kann eine Sicherheit erhöht werden, indem die Ventileinheit lediglich öffenbar ist, wenn die Transporteinheit mit der Applikatoreinheit verbunden wird.

Vorzugsweise weist die Ventileinheit ein Ventilelement auf, welches bevorzugt als Kugel ausgebildet ist. Das Ventilelement ist vorzugsweise aus Metall ausgebildet, wobei auch ein weiteres dem Fachmann sinnvoll erscheinendes Material, beispielsweise Kunststoff oder dergleichen, denkbar wäre. Die Ventileinheit weist vorzugsweise den Außenkörper der Transporteinheit zumindest teilweise auf. Vorzugsweise weist der, insbesondere zumindest abschnittsweise röhrenförmige, Außenkörper zumindest eine Verengung auf, in welcher das Ventilelement in einem geschlossenen Zustand der Ventileinheit angeordnet ist. Vorzugsweise ist das Ventilelement in dem geschlossenen Zustand der Ventileinheit formschlüssig und/oder reibschlüssig in der Verengung des Außenkörpers befestigt und verschließt insbesondere die zumindest eine Übertragungsöffnung. Vorzugsweise ist das Ventilelement zu einem Aufdrücken der Ventileinheit aus seiner befestigten Position, insbesondere bezüglich dem Außenkörper, verschiebbar. Vorzugsweise drückt ein Teil der Applikatoreinheit zu einem Aufdrücken der Ventileinheit direkt auf das Ventilelement. Alternativ wäre eine andere Ausgestaltung der Ventileinheit und insbesondere des Ventilelements denkbar, wobei die Ventileinheit beispielsweise durch einen Riegel oder dergleichen verschlossen werden könnte, welcher insbesondere beim Verbinden der Transporteinheit mit der Applikatoreinheit aufgedrückt wird. Vorzugsweise ist das Aufdrücken in eine dem Fluss der Flüssigkeitsprobe beim Übertrag der Flüssigkeitsprobe auf die Applikatoreinheit entgegengesetzte Richtung gerichtet.

Vorzugsweise ist die Ventileinheit lediglich von dem geschlossenen Zustand in einen aufgedrückten Zustand überführbar. Die Ventileinheit ist vorzugsweise einmalig aufdrückbar, wobei der aufgedrückte Zustand der Ventileinheit insbesondere auch nach einer Abnahme der Transporteinheit von der Applikatoreinheit nach dem Übertrag der Flüssigkeitsprobe bestehen bleibt. Vorzugsweise ist das Ventilelement in der aufgedrückten Ventileinheit formschlüssig und/oder reibschlüssig in einer, insbesondere von der Position des Ventilelements in der geschlossenen Ventileinheit unterschiedlichen, weiteren Position befestigt. Hierdurch kann ein Wiederverschluss der Ventileinheit und eine somit mögliche Mehrfachverwendung der, nach der ersten Anwendung kontaminierten, Transporteinheit besonders vorteilhaft vermieden werden. Alternativ wäre denkbar, dass das Ventilelement in der aufgedrückten Ventileinheit lose im Aufnahmebereich angeordnet ist. Alternativ wäre weiterhin denkbar, dass die Ventileinheit wiederverschließbar ist, wobei die Ventileinheit insbesondere lediglich durch ein aktives anhaltendes Aufdrücken durch die Applikatoreinheit im aufgedrückten Zustand haltbar sein könnte.

Insbesondere kann die Transporteinheit evakuierbar sein, sodass in ihrem Aufnahmebereich ein Unterdruck gegenüber einem Umgebungsdruck eingestellt werden kann. Hierzu weist die Transporteinheit vorzugsweise eine Vakuumventileinheit auf, die den Unterdruck im Aufnahmebereich nach einer Evakuierung hält. Die Evakuierung kann insbesondere vor oder während einer Einfüllung der Flüssigkeit in den Aufnahmebereich erfolgen. Insbesondere kann vorgesehen sein, dass die Vakuumventileinheit ein Vakuumventilelement aufweist, das bei Vorliegen eines Unterdrucks schließt. Vorzugsweise ist das Vakuumventilelement dazu vorgesehen, bei der Evakuierung geöffnet und mit Beendigung der Evakuierung geschlossen zu werden. Besonders bevorzugt ist das Vakuumventilelement dazu vorgesehen, durch den Umgebungsdruck in einem geschlossenen Zustand gehalten zu werden.

Ferner wird vorgeschlagen, dass die Applikatoreinheit zumindest eine Blockadeventileinheit aufweist, welche zumindest dazu vorgesehen ist, zumindest eine der Applikationen der Flüssigkeitsprobe wahlweise zu blockieren oder freizugeben. Dadurch kann vorteilhaft am Auslass des Applikators ein Austreten der Flüssigkeitsprobe, insbesondere von Blut, bei einem Befüllen der Kanäle, vorzugsweise unabhängig von einer Beschaffenheit, insbesondere Viskosität und Oberflächenspannung, verhindert werden. Vorzugsweise weist die Blockadeventileinheit zumindest ein Blockadeventil auf, welches zu einer wahlweisen Blockierung oder Freigabe öffenbar und/oder schließbar ausgestaltet ist. Insbesondere weist das erste Applikatorelement und/oder das zweite Applikatorelement zumindest das Blockadeventil der Blockadeventileinheit auf. Vorzugsweise ist das zumindest eine Blockadeventil in Strömungsrichtung betrachtet unmittelbar vor dem Kanalausgang, insbesondere am Ende des ersten Strömungskanals und/oder des zweiten Strömungskanals, angeordnet. Besonders bevorzugt ist die Blockadeventileinheit, insbesondere das zumindest eine Blockadeventil, dazu ausgestaltet, sich einem Zuklappen der Kartenträgerelemente automatisch zu schließen.

Zudem wird vorgeschlagen, dass das erste Applikatorelement und/oder das weitere Applikatorelement zumindest einen Entlüftungskanal zu einer Entlüftung zumindest einesder Strömungskanäle aufweist. Dadurch kann vorteilhaft bei geschlossenen Blockadeventilen ein Entweichen von Luft bereitgestellt werden. Ferner kann vorteilhaft ein einfaches Fließen der Flüssigkeitsprobe bis zu dem geschlossenen Ventil bereitgestellt werden. Insbesondere ist der Entlüftungskanal dazu vorgesehen, bei einem Einlass der Flüssigkeitsprobe in den ersten Strömungskanal und/oder den zweiten Strömungskanal bei zumindest einem geschlossenen Blockadeventil den jeweils ersten Strömungskanal und/oder den zweiten Strömungskanal zu entlüften. Insbesondere ist der Entlüftungskanal in Strömungsrichtung betrachtet, insbesondere unmittelbar, vor dem Blockadeventil angeordnet. Besonders bevorzugt verläuft der Entlüftungskanal zumindest im Wesentlichen in Richtung des Kanaleingangs, vorzugsweise in Richtung einer Oberseite, insbesondere bezüglich der Ausrichtung der Probenentnahmevorrichtung in zumindest dem Applikationszustand, insbesondere zumindest im Wesentlichen parallel zu einer Seitenkante des ersten Applikatorelements und/oder des zweiten Applikatorelements.

Zudem wird ein Verfahren zu einem Betrieb einer, insbesondere oben genannten Probenentnahmevorrichtung, wobei eine, insbesondere oben genannte, Flüssigkeitsprobe auf eine, insbesondere oben genannte, erste Probenkarte appliziert wird, wobei die Flüssigkeitsprobe in einem, insbesondere oben genannten, Applikationszustand gleichzeitig auf eine, insbesondere oben genannte, zweite Probenkarte appliziert wird, vorgeschlagen. Hierdurch kann eine Entnahmeeffizienz vorteilhaft gesteigert werden. Insbesondere kann die Probenentnahmevorrichtung vorteilhaft, insbesondere gleichzeitig, eine "A"- Probe auf der ersten Probenkarte und eine "B"-Probe auf der zweiten Probenkarte bereitstellen, beispielsweise für eine Doping-Kontrolle.

Das Verfahren weist vorzugsweise einen Verfahrensschritt, insbesondere einen Befestigungsschritt, auf, in welchem insbesondere die erste Probenkarte und die zweite Probenkarte an einer Kartenträgereinheit fixiert werden. Vorzugsweise weist das Verfahren einen weiteren, insbesondere zeitlich nach dem Befestigungsschritt angeordneten, Verfahrensschritt, insbesondere einen Entnahmeschritt auf, in welchem die Flüssigkeitsprobe in der Applikatoreinheit, insbesondere in dem ersten Strömungskanal und dem zweiten Strömungskanal, aufgenommen wird. Das Verfahren weist vorzugsweise einen weiteren Verfahrensschritt, insbesondere einen Kontaktierungsschritt auf, in welchem die Kartenträgereinheit zumindest teilweise gegenüber der Applikatoreinheit bewegt, insbesondere verschwenkt wird, und insbesondere ein Kontakt der ersten Probenkarte und der zweiten Probenkarte mit der Applikatoreinheit etabliert wird. Insbesondere ist der Kontaktierungsschritt zeitlich nach dem Entnahmeschritt angeordnet. Vorzugsweise weist das Verfahren einen weiteren, insbesondere zeitlich nach dem Kontaktierungsschritt angeordneten, Verfahrensschritt, insbesondere einen Applikationsschritt auf, in welchem die Flüssigkeitsprobe gleichzeitig auf die erste Probenkarte und die zweite Probenkarte appliziert wird. Das Verfahren kann einen weiteren Verfahrensschritt, insbesondere einen Herausnahmeschritt aufweisen, in welchem die Kartenträgereinheit gegenüber der Applikatoreinheit bewegt wird und insbesondere die erste Probenkarte und/oder die zweite Probenkarte, beispielsweise zu einem getrennten Transport und/oder einer getrennten Lagerung der ersten Probenkarte und der zweiten Probenkarte, aus der Kartenträgereinheit entnommen werden/wird. Der Herausnahmeschritt ist insbesondere zeitlich nach dem Applikationsschritt angeordnet.

### Zeichnungen

Weitere Vorteile ergeben sich aus der folgenden Zeichnungsbeschreibung. In den Zeichnungen sind drei Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine schematische Darstellung einer Probenentnahmevorrichtung mit einer Applikatoreinheit in einem Applikationszustand,
- Fig. 2: eine schematische Darstellung der Probenentnahmevorrichtung in einem weiteren Zustand,
- Fig. 3: eine schematische Darstellung der Applikatoreinheit,
- Fig. 4: eine Explosionsdarstellung der Applikatoreinheit,
- Fig. 5: eine schematische Darstellung eines ersten Applikatorelements der Applikatoreinheit,
- Fig. 6: eine schematische Darstellung eines zweiten Applikatorelements der Applikatoreinheit,
- Fig. 7: ein Ablaufdiagramm für ein Verfahren zu einem Betrieb der Probenentnahmevorrichtung,
- Fig. 8: eine schematische Darstellung eines alternativen Ausführungsbeispiels einer Probenentnahmevorrichtung mit einer von einer Applikationseinheit separaten Transporteinheit,
- Fig. 9: eine schematische Darstellung der Probenentnahmevorrichtung mit der mit der Applikatoreinheit verbundenen Transporteinheit,
- Fig. 10: eine Schnittansicht der Transporteinheit und
- Fig. 11: eine schematische Darstellung eines weiteren alternativen Ausführungsbeispiels einer Probeentnahmevorrichtung mit einer Applikationseinheit, die zumindest eine Blockadeventileinheit aufweist.

### Beschreibung der Ausführungsbeispiele

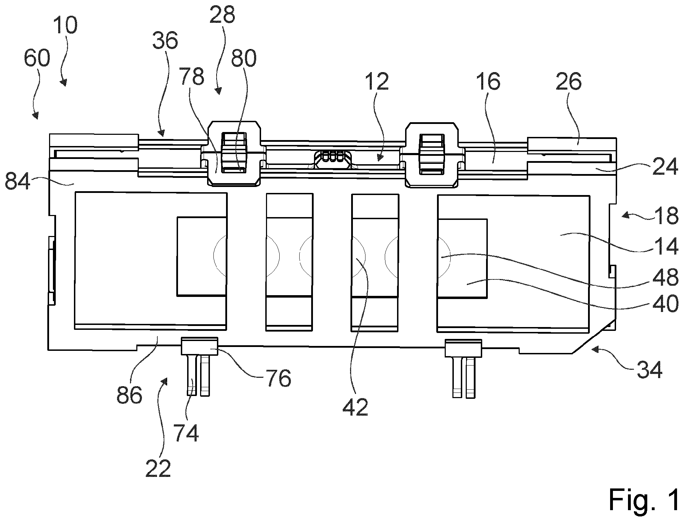

Figur 1 zeigt eine schematische Darstellung einer Probenentnahmevorrichtung 10 mit einer Applikatoreinheit 12 in einem Applikationszustand. Die Applikatoreinheit 12 ist in dem Applikationszustand zu einer gleichzeitigen Applikation einer Flüssigkeitsprobe (nicht dargestellt) auf eine erste Probenkarte 14 und eine zweite Probenkarte 16 vorgesehen.

Die Applikatoreinheit 12 ist in dem Applikationszustand zu einer Erzeugung eines ersten Probenspots (nicht dargestellt) auf einem Probenträger 40 der ersten Probenkarte 16 in einem vorliegend markierten Probenaufnahmebereich 48 des Probenträgers 40 vorgesehen. Der Probenträger 40 ist zu einer Absorption der Flüssigkeitsprobe vorgesehen. Der Probenträger 40 ist aus einem Filterpapier ausgebildet. Der Probenträger 40 weist zumindest einen weiteren Probenaufnahmebereich 42 auf. Entsprechendes gilt für die zweite Probenkarte 16, welche identisch zu der ersten Probenkarte 14 ausgebildet ist.

Die Probenentnahmevorrichtung 10 ist Teil eines Probenentnahmesystems 60, welches die ersten Probenkarte 14 und die zweiten Probenkarte 16 aufweist.

Die Probenentnahmevorrichtung 10 weist eine Kartenträgereinheit 18 auf, welche eine erste Halterung 24 für die erste Probenkarte 14 und eine zweite Halterung 26 für die zweite Probenkarte 16 aufweist. Die erste Halterung 24 ist Teil eines ersten Kartenträgerelements 34 der Kartenträgereinheit 18. Die zweite Halterung 26 ist Teil eines zweiten Kartenträgerelements 36 der Kartenträgereinheit 18. Die erste Halterung 24 und die zweite Halterung 26 sind zueinander identisch ausgebildet. Das erste Kartenträgerelement 34 und das zweite Kartenträgerelement 36 sind zueinander identisch ausgebildet. Im Folgenden soll lediglich das erste Kartenträgerelement 34 und die erste Halterung 24 beschrieben werden, wobei Entsprechendes für das zweite Kartenträgerelement 36 und die zweite Halterung 26 gilt.

Die erste Probenkarte 14 ist durch die erste Halterung 24 trennbar von dem ersten Kartenträgerelement 34 an dem ersten Kartenträgerelement 34 fixiert. Das erste Kartenträgerelement 34 weist ein Kartenträgerplattenelement 84 auf. Die erste Probenkarte 14 ist flach auf dem Kartenträgerplattenelement 84 angeordnet. Die erste Halterung 24 ist vorliegend aus einem abschnittsweisen Überhang an einem Rand des Kartenträgerplattenelements 84a ausgebildet. Die erste Probenkarte 14 ist innerhalb der Halterung 24 eingeklemmt.

Die Applikatoreinheit 12 ist zumindest in dem Applikationszustand mit der Kartenträgereinheit 18 verbunden. Die Applikatoreinheit 12 ist in dem Applikationszustand zumindest teilweise zwischen dem ersten Kartenträgerelement 34 und dem zweiten Kartenträgerelement 36 angeordnet. Die erste Probenkarte 14 ist in dem Applikationszustand zumindest teilweise zwischen der Applikatoreinheit 12 und dem ersten Kartenträgerelement 34 angeordnet. Die zweite Probenkarte 16 ist in dem Applikationszustand zumindest teilweise zwischen der Applikatoreinheit 12 und dem zweiten Kartenträgerelement 36 angeordnet. Die Applikatoreinheit 12, das erste Kartenträgerelement 34 und das zweite Kartenträgerelement 36 erstrecken sich in dem Applikationszustand parallel zueinander. Die Applikatoreinheit 12 ist in dem Applikationszustand zumindest zu einem Großteil zwischen dem ersten Kartenträgerelement 34 und dem zweiten Kartenträgerelement 36 angeordnet.

Die Probenentnahmevorrichtung 10 weist eine Fixiereinheit 22 auf, welche ein Herausrutschen der Applikatoreinheit 12 aus der Kartenträgereinheit 18 verhindert. Die Fixiereinheit 22 bildet eine formschlüssige Verbindung zwischen der Applikatoreinheit 12 und der Kartenträgereinheit 18 aus. Die Fixiereinheit 22 weist zumindest ein Applikatorhalterungselement 76 auf. Vorliegend weist die Fixiereinheit 22 vier zueinander identisch ausgebildete Applikatorhalterungselemente 76 auf, wobei lediglich ein Applikatorhalterungselement 76 bezeichnet ist. Die Applikatoreinheit 12 weist das Applikatorhalterungselement 76 auf. Ein Randbereich 86 der Kartenträgereinheit 18 ist zumindest teilweise formschlüssig in dem Applikatorhalterungselement 76 aufgenommen.

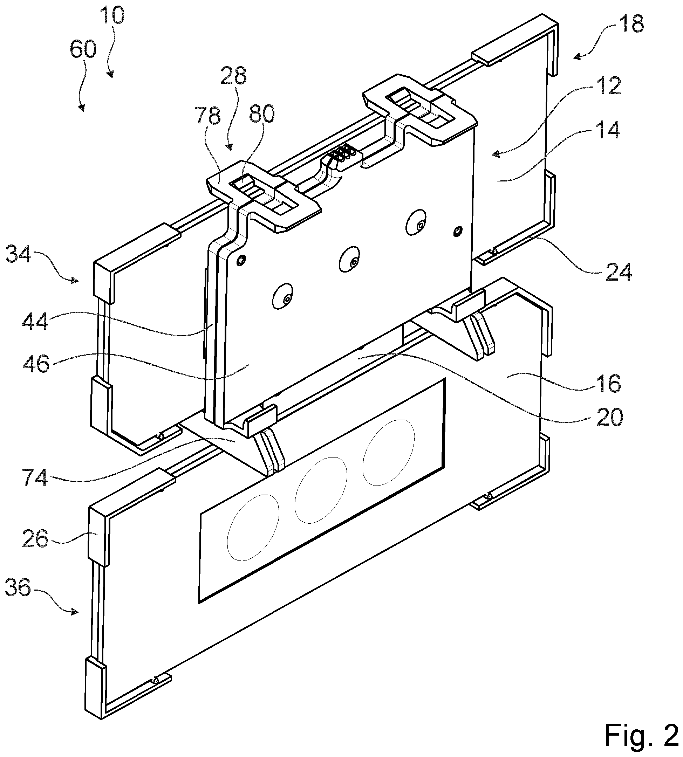

Figur 2 zeigt die Probenentnahmevorrichtung 10 in einem weiteren Zustand, welcher sich insbesondere von dem Applikationszustand und einem Entnahmezustand, in welchem die Applikatoreinheit 12 mit der Flüssigkeitsprobe befüllt wird, unterscheidet. Das zweite Kartenträgerelement 36 ist relativ zu dem ersten Kartenträgerelement 34 bewegbar. Vorliegend ist das zweite Kartenträgerelement 36 relativ zu dem ersten Kartenträgerelement 34 mittels eines Scharniers 20 der Kartenträgereinheit 18 verschwenkbar. Das erste Kartenträgerelement 34 und das zweite Kartenträgerelement 36 sind über das Scharnier 20 fest miteinander verbunden. Das Scharnier 20 ist für eine Verbindung des ersten Kartenträgerelements 34 und des zweiten Kartenträgerelements 36 vorgesehen, für welche in dem Applikationszustand eine gegenüberliegende Anordnung der ersten Probenkarte 14 und der zweiten Probenkarte 16 bereitgestellt ist (vgl. Figur 1).

Das erste Kartenträgerelement 34 und das zweite Kartenträgerelement 36 sind vorliegend einteilig miteinander verbunden. Das erste Kartenträgerelement 34 und das zweite Kartenträgerelement 36 sind vorliegend lediglich einteilig mittels des Scharniers 20 verbunden. Das Scharnier 20 ist vorliegend als ein Filmscharnier ausgebildet.

Die Probenentnahmevorrichtung 10 weist eine Verschlusseinheit 28, welche eine relative Lage des ersten Kartenträgerelements 34 zu dem zweiten Kartenträgerelement 36 in dem Applikationszustand fixiert, auf. Die Verschlusseinheit 28 bildet eine formschlüssige Verbindung zwischen der Applikatoreinheit 12 und der Kartenträgereinheit 18 aus. Die Verschlusseinheit 28 bildet vorliegend eine Schnappverbindung zwischen der Applikatoreinheit 12 und der Kartenträgereinheit 18 aus. Die Verschlusseinheit 28 weist zumindest ein Verschlusselement 78 auf. Die Applikatoreinheit 12 weist das Verschlusselement 78 auf. Die Verschlusseinheit 28 weist zumindest ein Gegenverschlusselement 80 auf. Die Kartenträgereinheit 18 weist das Gegenverschlusselement 80 auf. Das Gegenverschlusselement 80 der Kartenträgereinheit 18 ist zumindest in dem Applikationszustand zumindest teilweise formschlüssig in dem Verschlusselement 78 aufgenommen (vgl. Figur 1). Vorliegend weist die Verschlusseinheit 28 vier zueinander identisch ausgebildete Verschlusselemente 78 und vier zueinander identisch ausgebildete Gegenverschlusselemente 80 auf, wobei lediglich ein Verschlusselement 78 und lediglich ein Gegenverschlusselement 80 bezeichnet sind

Die Applikatoreinheit 12 ist in dem Applikationszustand mittels der Fixiereinheit 22 und der Verschlusseinheit 28 unbeweglich mit der Kartenträgereinheit 18 verbunden (vgl. Figur 1).

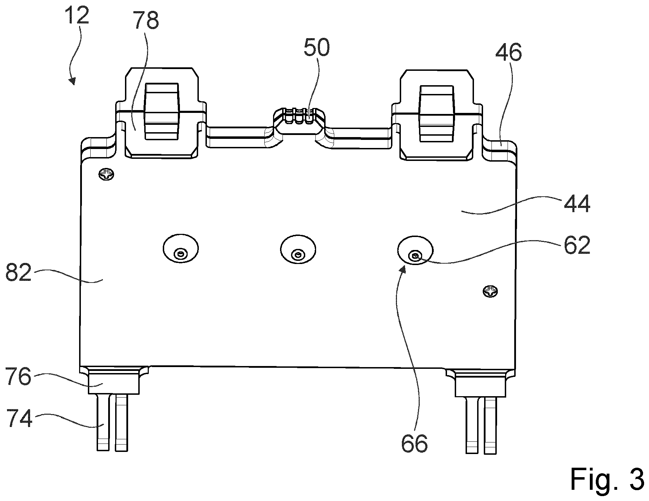

Figur 3 zeigt die Applikatoreinheit 12 vergrößert. Die Applikatoreinheit 12 weist ein erstes Applikatorelement 44 zu der Applikation der Flüssigkeitsprobe auf die erste Probenkarte 14 und ein zweites Applikatorelement 46 zu der Applikation der Flüssigkeitsprobe auf die zweite Probenkarte 16 auf. Das erste Applikatorelement 44 und das zweite Applikatorelement 46 sind zueinander identisch ausgebildet. Es soll folgend lediglich das erste Applikatorelement 44 beschrieben werden, wobei Entsprechendes für das zweite Applikatorelement 46 gilt. Das Applikatorelement 46 weist zumindest das Applikatorhalterungselement 76, das Verschlusselement 78, ein Applikatorplattenelement 82 und einen Standfuß 74 auf. Die Applikatoreinheit 12 weist vorliegend vier Standfüße 74 auf, wobei lediglich einer bezeichnet ist. Die Probenentnahmevorrichtung 10 wird zumindest in dem Applikationszustand mittels der Standfüße 74 aufgestellt (vgl. Figur 1).

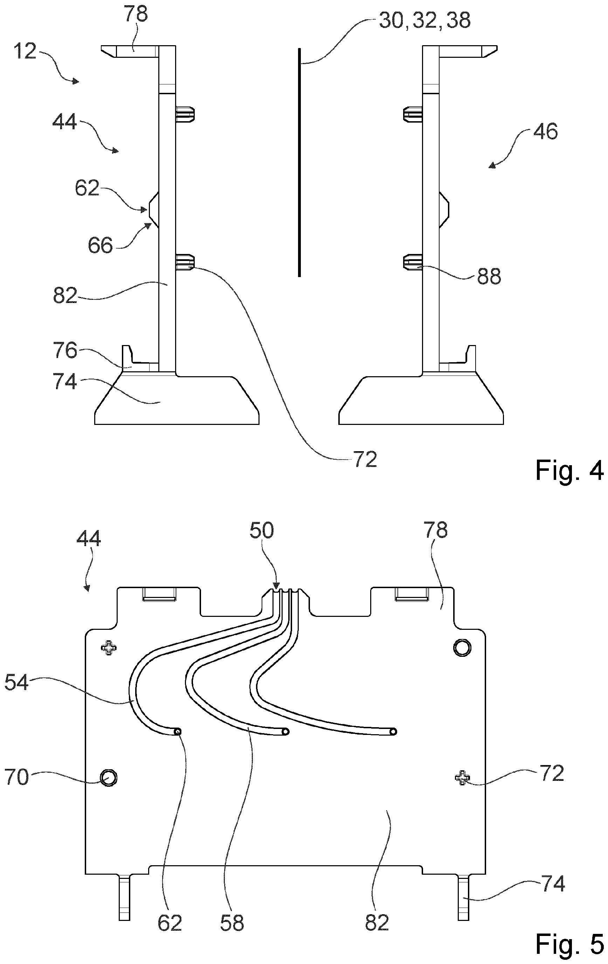

Figur 4 zeigt eine Explosionsdarstellung der Applikatoreinheit 12. Das erste Applikatorelement 44 und das zweite Applikatorelement 46 sind über eine Trenneinheit 30 der Applikatoreinheit 12 miteinander verbunden. Die Trenneinheit 30 ist vorliegend als eine Folieneinheit 32 mit einer doppelklebenden Folie 38 ausgebildet.

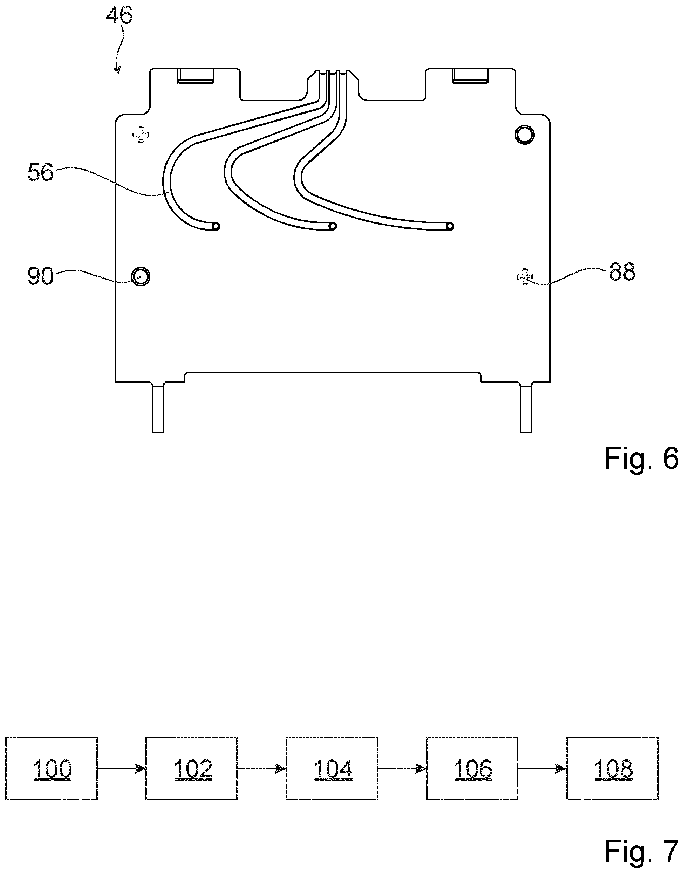

Das erste Applikatorelement 44 ist zudem über eine Steckverbindung (nicht dargestellt) mit dem zweiten Applikatorelement 46 verbunden. Figur 5 zeigt das erste Applikatorelement 44a. Das erste Applikatorelement 44 weist ein Steckelement 72 auf, welches zu einer reibschlüssigen Verbindung des ersten Applikatorelements 44 mit dem zweiten Applikatorelement 46 zu einer Aufnahme in einer Ausnehmung 90 des zweiten Applikatorelements 46 vorgesehen ist (vgl. Figur 6). Das zweite Applikatorelement 46 weist ein Steckelement 88 auf, welches zu einer reibschlüssigen Verbindung des ersten Applikatorelements 44 mit dem zweiten Applikatorelement 46 zu einer Aufnahme in einer Ausnehmung 70 des ersten Applikatorelements 44 vorgesehen ist.

Die erste Applikatorelement 44 weist zumindest einen ersten Strömungskanal 54 und das zweite Applikatorelement 46 weist zumindest einen zweiten Strömungskanal 56 (vgl. Figur 6) auf, wobei der erste Strömungskanal 54 und der zweite Strömungskanal 56 durch die Trenneinheit 30 (vgl. Figur 4) zumindest teilweise verschlossen sind. Der erste Strömungskanal 54 und der zweite Strömungskanal 56 ist zu einem Transport der Flüssigkeitsprobe zu der Applikation auf die erste Probenkarte 14 und die zweite Probenkarte 16 vorgesehen. Der erste Strömungskanal 54 ist identisch zu dem zweiten Strömungskanal 56 ausgebildet. Im Folgenden soll lediglich das erste Applikatorelement 44 und der erste Strömungskanal 54 beschrieben werden, wobei Entsprechendes für das zweite Applikatorelement 46 und den zweiten Strömungskanal 56 gilt.

Der erste Strömungskanal 54 weist einen Kanaleingang 50 auf, an welchen eine Flüssigkeitsquelle (nicht dargestellt) der Flüssigkeitsprobe zu einem Befüllen des ersten Strömungskanals 54 mit der Flüssigkeitsprobe angelegt wird. Der erste Strömungskanal 54 weist einen unterhalb dem Kanaleingang 50 angeordneten Kanalausgang 62 auf. Der Kanalausgang 62 ist in einer Ausbuchtung 66 des ersten Applikatorelements 44 ausgebildet. Die Ausbuchtung 66 des ersten Applikatorelements 44 ist zu einer Kontaktierung des Kanalausgangs 62 mit der ersten Probenkarte 14 vorgesehen (vgl. Figur 2). Der erste Strömungskanal 54 ist zu einer Aufnahme eines quantifizierten Volumens der Flüssigkeitsprobe ausgebildet. Der erste Strömungskanal 54 ist gekrümmt ausgebildet. Das erste Applikatorelement 44 weist zumindest einen weiteren ersten Strömungskanal 58 auf, welcher ein zu dem ersten Strömungskanal 54 identisches Volumen der Flüssigkeitsprobe fasst. Der weitere erste Strömungskanal 58 ist zu einer Erzeugung eines weiteren Probenspots (nicht dargestellt) in dem weiteren Probenaufnahmebereich 42 vorgesehen (vgl. Figur 1)

Figur 6 zeigt das zweite Applikatorelement 46.

Figur 7 zeigt ein Ablaufdiagramm eines Verfahrens zu einem Betrieb der Probenentnahmevorrichtung 10. Das Verfahren weist einen Befestigungsschritt 100 auf, in welchem die erste Probenkarte 14 und die zweite Probenkarte 16 an der Kartenträgereinheit 18 fixiert werden. In einem Entnahmeschritt 102 des Verfahrens wird die Flüssigkeitsprobe in der Applikatoreinheit 12 aufgenommen. Das Verfahren weist einen Kontaktierungsschritt 104 auf, in welchem die Kartenträgereinheit 18 gegenüber der Applikatoreinheit 12 verschwenkt wird. In dem Kontaktierungsschritt 104 wird jeweils ein Kontakt der ersten Probenkarte 14 und der zweiten Probenkarte 16 mit der Applikatoreinheit 12 etabliert. Die Flüssigkeitsprobe wird in einem Applikationsschritt 106 des Verfahrens gleichzeitig auf die erste Probenkarte 14 und die zweite Probenkarte 16 appliziert. Das Verfahren weist einen Herausnahmeschritt 108 auf, in welchem die erste Probenkarte 14 und die zweite Probenkarte 16, beispielsweise zu einem getrennten Transport und/oder einer getrennten Lagerung, aus der Probenentnahmevorrichtung 10 entnommen werden.

In einer nicht erfindungsgemäßen Anwendung für die Medizin wäre denkbar, dass das erste Applikatorelement 44 oder das zweite Applikatorelement 46 als eine Blindplatte ohne Kanäle ausgebildet ist.

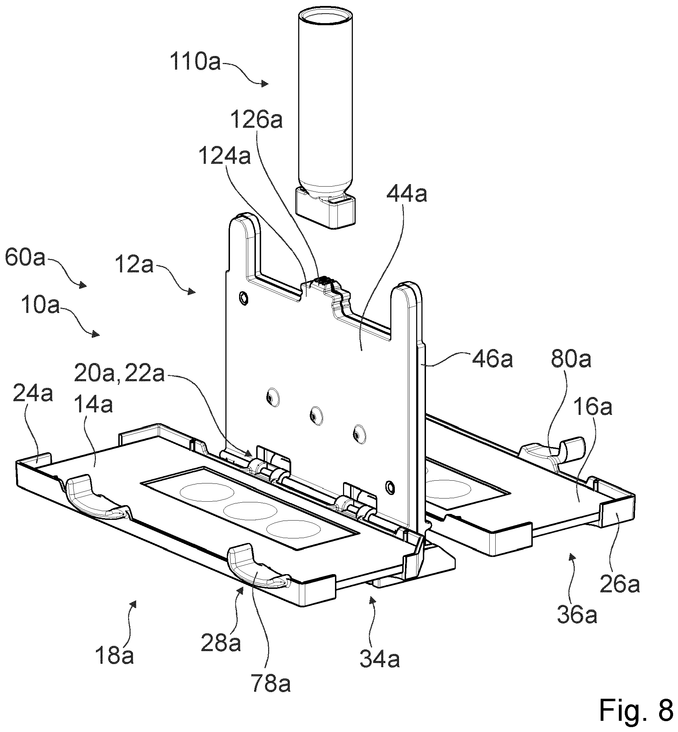

In den Figuren 8 bis 11 sind zwei weitere Ausführungsbeispiele der Erfindung gezeigt. Die nachfolgenden Beschreibungen und die Zeichnungen beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnungen und/oder die Beschreibung des anderen Ausführungsbeispiels, insbesondere der Figuren 1 bis 7, verwiesen werden kann. Zur Unterscheidung der Ausführungsbeispiele sind die Buchstaben a und b den Bezugszeichen der Ausführungsbeispiele in den Figuren 8 bis 11 nachgestellt.

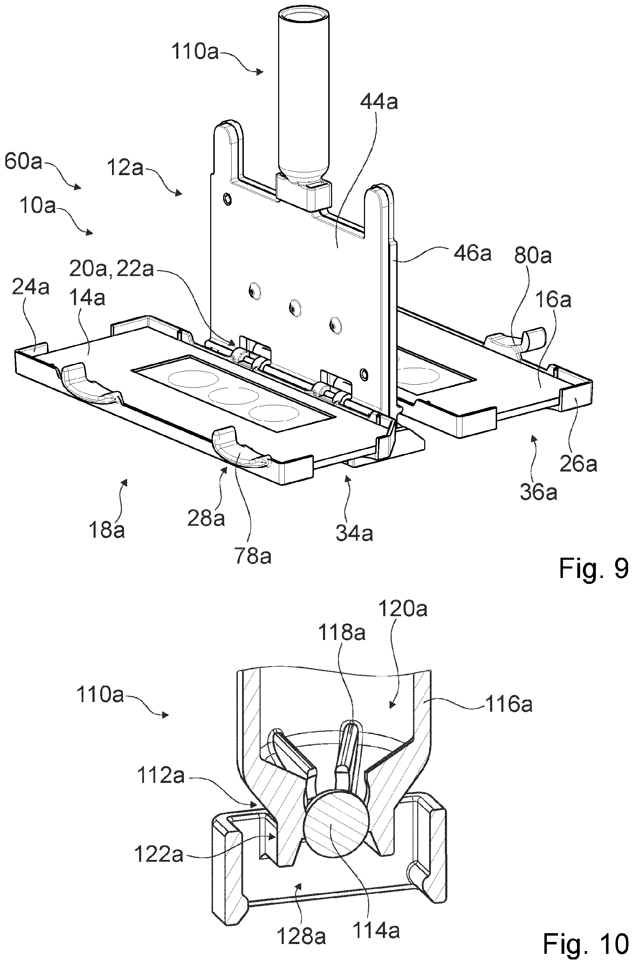

Figur 8 zeigt eine Probenentnahmevorrichtung 10a eines Probenentnahmesystems 60a. Die Probenentnahmevorrichtung 10a weist eine Transporteinheit 110a auf, welche zu einer Übertragung einer Flüssigkeitsprobe (nicht dargestellt) an eine Applikatoreinheit 12a mit dieser verbindbar ist (vgl. Figur 9). Die Transporteinheit 110a ist zu einem Transport der Flüssigkeitsprobe zwischen einer Flüssigkeitsquelle (nicht dargestellt) und der Applikatoreinheit 12a vorgesehen. Die Transporteinheit 110a ist abschnittsweise röhrenförmig ausgebildet.

Ein erstes Kartenträgerelement 34a ist vorliegend über die Applikatoreinheit 12a mit einem zweiten Kartenträgerelement 36a verbunden. Das erste Kartenträgerelement 34a und das zweite Kartenträgerelement 36a sind jeweils über ein Scharnier 20a mit der Applikatoreinheit 12a verbunden. Eine Fixiereinheit 22a ist einstückig mit dem Scharnier 20a ausgebildet.

Das erste Kartenträgerelement 34a weist ein Verschlusselement 78a auf. Das zweite Kartenträgerelement 36a weist ein Gegenverschlusselement 80a auf. Eine Verschlusseinheit 28a mit dem Verschlusselement 78a und dem Gegenverschlusselement 80a sind zur Fixierung der relativen Lage des ersten Kartenträgerelements 34a zu dem zweiten Kartenträgerelement 36a in dem Applikationszustand vorgesehen. Die Verschlusseinheit 28a funktioniert vorliegend entsprechend einem Schnappverschluss.

Die Applikatoreinheit 12a weist einen Fortsatz 124a auf. Der Fortsatz 124a steht bezüglich einer flachen oberen Kante der Applikatoreinheit 12a über. Der Fortsatz 124a weist ein Betätigungselement 126a auf. Das Betätigungselement 126a ist oberhalb dem restlichen Fortsatz 124a angeordnet. Das Betätigungselement 126a ist bezüglich zumindest einer Ansichtsrichtung schmaler als der restliche Fortsatz 124a ausgebildet. Das Betätigungselement 126a weist zumindest einen Kanaleingang (nicht dargestellt) auf.

Figur 9 zeigt die Probenentnahmevorrichtung 10a in einem Entnahmezustand. Die Transporteinheit 110a ist zumindest in dem Entnahmezustand mit der Applikatoreinheit 12a verbunden. Die Transporteinheit 110a ist in dem Entnahmezustand flüssigkeitsdicht mit der Applikatoreinheit 12a verbunden. Die Transporteinheit 110a ist formschlüssig und reibschlüssig mit der Applikatoreinheit 12a verbindbar. Die Transporteinheit 110a ist zur Übertragung der Flüssigkeitsprobe an die Applikatoreinheit 12a auf der Applikatoreinheit 12a durch Aufdrücken aufgesteckt.

Figur 10 zeigt eine Schnittdarstellung der Transporteinheit 110a in einem Zustand entsprechend Figur 8. Die Transporteinheit 110a weist eine Ventileinheit 112a auf, welche dazu vorgesehen ist, beim Verbinden der Transporteinheit 110a mit der Applikatoreinheit 12a durch die Applikatoreinheit 12a aufgedrückt zu werden.

Die Ventileinheit 112a weist ein Ventilelement 114a auf. Das Ventilelement 114a ist vorliegend als eine Kugel aus Metall ausgebildet. Die Transporteinheit 110a weist einen Außenkörper 116a auf. Der Außenkörper 116a ist abschnittsweise röhrenförmig ausgebildet und begrenzt einen Aufnahmebereich 120a für die Flüssigkeitsprobe. Die Ventileinheit 112a weist zumindest einen Teil des Außenkörpers 116a auf.

Das Ventilelement 114a ist in einem geschlossenen Zustand der Ventileinheit 112a formschlüssig in einer Verengung 122a in dem Außenkörper 116a der Transporteinheit 110a gelagert. Der Außenkörper 116a weist vorliegend Ventilausbuchtungen 118a auf, wobei lediglich eine Ventilausbuchtung 118a bezeichnet ist. Das Ventilelement 114a ist durch die Applikatoreinheit 12a zwischen die Ventilausbuchtungen 118a drückbar. Die Flüssigkeitsprobe ist zwischen den Ventilausbuchtungen 118a an die Applikatoreinheit 12a übertragbar. Die Ventilausbuchtung 118a ist vorliegend einteilig mit dem Außenkörper 116a ausgebildet.

Die Transporteinheit 110a weist einen Applikatoreinheitaufnahmeraum 128a auf, welcher zu einer Übertragung der Flüssigkeitsprobe dazu vorgesehen ist, einen Teil der Applikatoreinheit 12a aufzunehmen. Der Applikatoreinheitaufnahmeraum 128a ist zu einer Übertragung der Flüssigkeitsprobe dazu vorgesehen den Fortsatz 124a aufzunehmen (vgl. Figur 8). Der Applikatoreinheitaufnahmeraum 128a ist zumindest teilweise durch den Außenkörper 116a begrenzt. Eine Form des Applikatoreinheitaufnahmeraums 128a entspricht zumindest im Wesentlichen einer Form des Fortsatzes 124a.

Das Betätigungselement 126a ist zum Aufdrücken der Ventileinheit 112a zu einem Kontakt mit dem Ventilelement 114a vorgesehen.

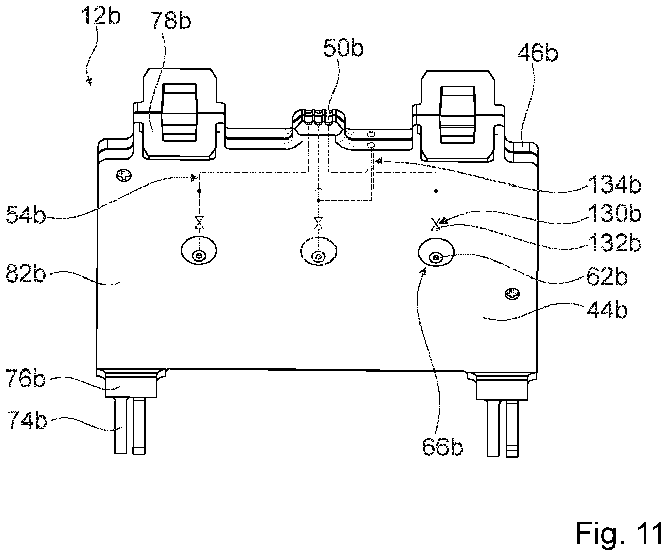

Die Figur 11 zeigt eine weitere Probenentnahmevorrichtung 10b eines Probenentnahmesystems 60b. Die Probenentnahmevorrichtung 10b weist eine Applikatoreinheit 12b auf. Die Applikatoreinheit 12b weist zumindest eine Blockadeventileinheit 130b auf. Die Blockadeventileinheit 130b ist dazu vorgesehen, zumindest eine von zumindest zwei Applikationen einer Flüssigkeitsprobe wahlweise zu blockieren oder freizugeben. Die Blockadeventileinheit 130b weist zumindest ein Blockadeventil 132b auf. Die Applikatoreinheit 12b weist zumindest ein erstes Applikatorelement 44b und ein zweites Applikatorelement 46b auf. Das erste Applikatorelement 44b und/oder das zweite Applikatorelement 46b weist zumindest das Blockadeventil 132b der Blockadeventileinheit 130b auf. Das erste Applikatorelement 44b und das zweite Applikatorelement 46b weisen jeweils zumindest einen ersten Strömungskanal 54b und zumindest einen zweiten Strömungskanal 56b auf. Das zumindest eine Blockadeventil 132b ist in Strömungsrichtung betrachtet unmittelbar vor einem Kanalausgang 62b des ersten Strömungskanals 54b und/oder des zweiten Strömungskanals 56b angeordnet. Das zumindest eine Blockadeventil 132b ist dazu vorgesehen, bei einem Zuklappen von Kartenträgerelemente 34b, 36b automatisch zu schließen. Alternativ oder zusätzlich weist die Applikatoreinheit 12b zumindest ein Betätigungselement auf, welches das Blockadeventil 132b wahlweise öffnet und/oder schließt. Das Applikatorelement 12b weist zumindest drei erste Strömungskanäle 54b und/oder zweite Strömungskanäle 56b auf, wobei jeder der Strömungskanäle 54b, 56b ein Blockadeventil 132b an dem Kanalausgang 62b aufweist.

Das erste Applikatorelement 44b und/oder das zweite Applikatorelement 46b weist zumindest einen Entlüftungskanal 134b auf. Der Entlüftungskanal 134b ist zu einer Entlüftung zumindest eines der Strömungskanäle 54b, 56b vorgesehen. Der Entlüftungskanal 134b ist dazu vorgesehen, bei einem Einlass der Flüssigkeitsprobe in den ersten Strömungskanal 54b und/oder den zweiten Strömungskanal 56b bei zumindest einem geschlossenen Blockadeventil 132b, den jeweils ersten Strömungskanal 54b und/oder den zweiten Strömungskanal 56b zu entlüften. Der Entlüftungskanal 134b ist in Strömungsrichtung betrachtet unmittelbar vor dem Blockadeventil 132b angeordnet. Der Entlüftungskanal 134b verläuft zumindest im Wesentlichen in Richtung eines Kanaleingangs 50b. Der Entlüftungskanal 134b verläuft in Richtung einer Oberseite der Applikatoreinheit 12b bezüglich der Ausrichtung der Probenentnahmevorrichtung 10b in zumindest einem Applikationszustand. Das erste Applikatorelement 44b und/oder das Applikatorelement 46b weist mehrere Entlüftungskanäle 134b auf, wobei jeder erste Strömungskanal 54b und/oder jede zweite Strömungskanal 56b jeweils einen Entlüftungskanal 134b aufweist. Die Entlüftungskanäle 134b laufen zu einer Entlüftungsöffnung zusammen. Alternativ weist jeder Entlüftungskanal 134b eine eigene Entlüftungsöffnung auf.

### Bezugszeichen

- 10: Probenentnahmevorrichtung
- 12: Applikatoreinheit
- 14: erste Probenkarte
- 16: zweite Probenkarte
- 18: Kartenträgereinheit
- 20: Scharnier
- 22: Fixiereinheit
- 24: erste Halterung
- 26: zweite Halterung
- 28: Verschlusseinheit
- 30: Trenneinheit
- 32: Folieneinheit
- 34: erstes Kartenträgerelement
- 36: zweites Kartenträgerelement
- 38: Folie
- 40: Probenträger
- 42: weiterer Probenaufnahmebereich
- 44: erstes Applikatorelement
- 46: zweites Applikatorelement
- 48: Probenaufnahmebereich
- 50: Kanaleingang
- 54: erster Strömungskanal
- 56: zweiter Strömungskanal
- 58: weiterer erster Strömungskanal
- 60: Probenentnahmesystem
- 62: Kanalausgang
- 66: Ausbuchtung
- 70: Ausnehmung
- 72: Steckelement
- 74: Standfuß
- 76: Applikatorhalterungselement
- 78: Verschlusselement
- 80: Gegenverschlusselement
- 82: Applikatorplattenelement
- 84: Kartenträgerplattenelement
- 86: Randbereich
- 88: Steckelement
- 90: Ausnehmung
- 100: Befestigungsschritt
- 102: Entnahmeschritt
- 104: Kontaktierungsschritt
- 106: Applikationsschritt
- 108: Herausnahmeschritt
- 110: Transporteinheit
- 112: Ventileinheit
- 114: Ventilelement
- 116: Außenkörper
- 118: Ventilausbuchtung
- 120: Aufnahmebereich
- 122: Verengung
- 124: Fortsatz
- 126: Betätigungselement
- 128: Applikatoreinheitaufnahmeraum
- 130: Blockadeventileinheit
- 132: Blockadeventil
- 134: Entlüftungskanal

## Patentansprüche

1. Probenentnahmevorrichtung (10; 10a; 10b) mit einer Applikatoreinheit (12; 12a, 12b) zu einer Applikation einer Flüssigkeitsprobe auf eine erste Probenkarte (14; 14a; 14b), **dadurch gekennzeichnet, dass** die Applikatoreinheit (12; 12a; 12b) in einem Applikationszustand zu einer gleichzeitigen Applikation der Flüssigkeitsprobe auf eine zweite Probenkarte (16; 16a; 16b) vorgesehen ist.

2. Probenentnahmevorrichtung (10; 10a; 10b) nach Anspruch 1, **gekennzeichnet durch** eine Kartenträgereinheit (18; 18a, 18b), welche eine erste Halterung (24; 24a; 24b) für die erste Probenkarte (14; 14a; 14b) und eine zweite Halterung (26; 26a; 26b) für die zweite Probenkarte (16; 16a; 16b) aufweist.

3. Probenentnahmevorrichtung (10; 10a; 10b) nach Anspruch 2, **dadurch gekennzeichnet, dass** die Kartenträgereinheit (18; 18a; 18b) ein erstes Kartenträgerelement (34; 34a; 34b), welches die erste Halterung (24; 24a; 24b) aufweist, und ein relativ zu dem ersten Kartenträgerelement (34; 34a; 34b) bewegbares zweites Kartenträgerelement (36; 36a; 36b), welches die zweite Halterung (26; 26a; 26b) aufweist, umfasst.

4. Probenentnahmevorrichtung (10; 10a; 10b) nach Anspruch 3, **dadurch gekennzeichnet, dass** das zweite Kartenträgerelement (36; 36a; 36b) relativ zu dem ersten Kartenträgerelement (34; 34a; 34b) mittels zumindest eines Scharniers (20; 20a; 20b) der Kartenträgereinheit (18; 18a; 18b) verschwenkbar ist.

5. Probenentnahmevorrichtung (10; 10a; 10b) nach dem Anspruch 3 oder 4, **dadurch gekennzeichnet, dass** das erste Kartenträgerelement (34; 34a; 34b) und das zweite Kartenträgerelement (36; 36a; 36b) fest, insbesondere einteilig, miteinander verbunden sind.

6. Probenentnahmevorrichtung (10; 10a; 10b) nach einem der Ansprüche 2 bis 5, **dadurch gekennzeichnet, dass** die Applikatoreinheit (12; 12a; 12b) zumindest in dem Applikationszustand mit der Kartenträgereinheit (18; 18a; 18b) verbunden ist.

7. Probenentnahmevorrichtung (10; 10a; 10b) nach Anspruch 6, **dadurch gekennzeichnet, dass** die Applikatoreinheit (12; 12a; 12b) in dem Applikationszustand zumindest teilweise zwischen dem ersten Kartenträgerelement (34; 34a; 34b) und dem zweiten Kartenträgerelement (36; 36a; 36b) angeordnet ist.

8. Probenentnahmevorrichtung (10; 10a; 10b) nach Anspruch 7, **gekennzeichnet durch** eine Fixiereinheit (22; 22a; 22b), welche ein Herausrutschen der Applikatoreinheit (12; 12a; 12b) aus der Kartenträgereinheit (18; 18a; 18b) verhindert.

9. Probenentnahmevorrichtung (10; 10a; 10b) nach einem der Ansprüche 3 bis 8, **gekennzeichnet durch** eine Verschlusseinheit (28; 28a; 28b), welche eine relative Lage des ersten Kartenträgerelements (34; 34a; 34b) zu dem zweiten Kartenträgerelement (36; 36a; 36b) in dem Applikationszustand fixiert.

10. Probenentnahmevorrichtung (10; 10a; 10b) nach einem der vorhergehenden Ansprüche, **dadurch gekennzeichnet, dass** die Applikatoreinheit (12; 12a; 12b) ein erstes Applikatorelement (44; 44a; 44b) zu einer Applikation der Flüssigkeitsprobe auf die erste Probenkarte (14; 14a; 14b) und ein zweites Applikatorelement (46; 46a; 46b) zu einer Applikation der Flüssigkeitsprobe auf die zweite Probenkarte (16; 16a; 16b) aufweist, wobei das erste Applikatorelement (44; 44a; 44b) und das zweite Applikatorelement (46; 46a; 46b) über eine Trenneinheit (30; 30a; 30b), insbesondere eine Folieneinheit (32; 32a; 32b), der Applikatoreinheit (12; 12a; 12b) verbunden sind.

11. Probenentnahmevorrichtung (10; 10a; 10b) nach Anspruch 10, **dadurch gekennzeichnet, dass** das erste Applikatorelement (44; 44a; 44b) zumindest einen ersten Strömungskanal (54; 54a; 54b) und das zweite Applikatorelement (46; 46a; 46b) zumindest einen zweiten Strömungskanal (56; 56a; 56b) aufweist, wobei der erste Strömungskanal (54; 54a; 54b) und der zweite Strömungskanal (56; 56a; 56b) durch die Trenneinheit (30; 30a; 30b) zumindest teilweise verschlossen sind.

12. Probenentnahmevorrichtung (10; 10a; 10b) nach Anspruch 10 oder 11, **dadurch gekennzeichnet, dass** das erste Applikatorelement (44; 44a; 44b) und das zweite Applikatorelement (46; 46a; 46b) zueinander identisch ausgebildet sind.

13. Probenentnahmevorrichtung (10a) zumindest nach dem Oberbegriff des Anspruchs 1 und insbesondere nach einem der vorhergehenden Ansprüche, **gekennzeichnet durch** eine Transporteinheit (110a), welche zu einer Übertragung der Flüssigkeitsprobe an die Applikatoreinheit (12a) mit dieser verbindbar ist.

14. Probenentnahmevorrichtung (10a) nach Anspruch 13, **dadurch gekennzeichnet, dass** die Transporteinheit (110a) eine Ventileinheit (112a) aufweist, welche dazu vorgesehen ist, beim Verbinden der Transporteinheit (110a) mit der Applikatoreinheit (12a) durch die Applikatoreinheit (12a) aufgedrückt zu werden.

15. Probenentnahmevorrichtung (10b) nach einem der vorhergehenden Ansprüche, **dadurch gekennzeichnet, dass** die Applikatoreinheit (12b) zumindest eine Blockadeventileinheit (130b) aufweist, welche zumindest dazu vorgesehen ist, zumindest eine der Applikationen der Flüssigkeitsprobe wahlweise zu blockieren oder freizugeben.

16. Probeentnahmevorrichtung (10b) nach Anspruch 10, **dadurch gekennzeichnet, dass** das erste Applikatorelement (44b) und/oder das zweite Applikatorelement (46b) zumindest einen Entlüftungskanal (134b) zu einer Entlüftung zumindest eines der Strömungskanäle (54b, 56b) aufweist.

17. Probenentnahmesystem (60; 60a; 60b) mit einer Probenentnahmevorrichtung (10; 10a; 10b) nach einem der vorhergehenden Ansprüche, mit der ersten Probenkarte (14; 14a; 14b) und mit der zweiten Probenkarte (16; 16a; 16b).

18. Verfahren zu einem Betrieb einer Probenentnahmevorrichtung (10; 10a; 10b), insbesondere nach einem der Ansprüche 1 bis 16, wobei eine Flüssigkeitsprobe auf eine erste Probenkarte (14; 14a; 14b) appliziert wird, **dadurch gekennzeichnet, dass** die Flüssigkeitsprobe in einem Applikationszustand gleichzeitig auf eine zweite Probenkarte (16; 16a; 16b) appliziert wird.
